# EUROPÄISCHE PATENTSCHRIFT

(11) **EP 1 104 318 B1**
(45) Veröffentlichungstag und Bekanntmachung des Hinweises auf die Patenterteilung: **11.09.2002**
(21) Anmeldenummer: 99944387.2
(22) Anmeldetag: 13.08.1999
(51) Int. Cl.: A61M 15/00, G06M 1/08, G06M 1/14

(54) **INHALATOR MIT EINER DOSIERZÄHLEINRICHTUNG**
INHALATOR COMPRISING A DOSAGE COUNTING DEVICE
INHALATEUR A SYSTEME DE COMPTAGE DES DOSES

(30) Priorität: 14.08.1998 DE 29814647 U
(43) Veröffentlichungstag der Anmeldung: 06.06.2001
(73) Patentinhaber: RPC Wiko GmbH & Co. KG, 50259 Pulheim (DE)
(72) Erfinder: ECKERT, Joseph, D-97638 Mellrichstadt (DE)
(74) Vertreter: Mey, Klaus-Peter, Dr.-Ing. Dipl.-Wirtsch.-Ing.
(86) Internationale Anmeldenummer: EP9905939
(87) Internationale Veröffentlichungsnummer: WO00009187

(56) Entgegenhaltungen:
- EP-A- 0 480 488
- EP-A- 0 949 584
- WO-A-94/14492
- WO-A-96/29278
- FR-A- 2 022 212

## Beschreibung

Die Erfindung betrifft einen Inhalator zur Abgabe eines Aerosols mit einer Dosierzähleinrichtung, einem Gehäuseteil und einem relativ zu dem Gehäuseteil entlang seiner Längsachse verschiebbar angeordneten Aerosol-Abgabebehälter.

Ein derartiger Inhalator ist aus EP-A1-0 254 391 bekannt. Bei diesem herkömmlichen Inhalator ist ein Aerosol-Abgabebehälter verschiebbar in einen rohrförmigen Abschnitt eines Gehäuseteils eingesetzt. Der Abgabebehälter umfaßt ein aus einem Kopfbereich des Abgabebehälters herausgeführtes Sprührohr, das in abdichtender Weise in einen Sprühsockelabschnitt eingesetzt ist, der einstückig mit dem Gehäuse ausgebildet ist. Zur Abgabe einer festgelegten Dosis eines in dem Abgabebehälter bevorrateten Mediums wird auf einen Bodenabschnitt des Abgabebehälters eine Druckkraft ausgeübt und der Behälter kurzzeitig in das Gehäuse hineingedrängt. Über das in den Sprühsockel eingesetzte Sprührohr wird kurzzeitig ein Ventilmechanismus geöffnet und eine vorbestimmte Menge des in dem Abgabebehälter bevorrateten Mediums aus dem Sprühkopf abgesprüht. In einem Seitenbereich des Gehäuses ist ein drehbewegbar gelagertes Anzeigerad vorgesehen, das bei jedem Abgabehub des Inhalators über ein Untersetzungsgetriebe um einen geringen Drehbetrag weitergedreht wird. Das Anzeigerad ist mit mehreren Füllstandsangaben versehen, die bei sukzessiver Drehung des Anzeigerades über einen Fensterabschnitt ablesbar sind. Wenn der Abgabebehälter nahezu vollständig entleert ist, wird in dem Fenster ein entsprechendes Symbol sichtbar und dem Benutzer damit signalisiert, daß der Abgabebehälter im wesentlichen vollständig entleert ist. Durch eine werkseitig vorgenommene Überfüllung des Abgabebehälters um ca. 10 bis 15 % ist gewährleistet, daß bei Erreichen dieser End-Markierung noch eine ausreichende Wirkstoffmenge in dem Abgabebehälter bevorratet ist.

Diese werkseitig vorgenommene Überfüllung des Abgabebehälters fuhrt insbesondere bei der Abfüllung von vergleichsweise teuren Wirkstoffen zu einer Verteuerung des Inhalators. Auch unter Umweltverträglichkeitsaspekten erscheint eine Überfüllung des Abgabebehälters im bislang üblichen Umfang als problematisch.

Ein weiteres Beispiel der herrkömmlichen Inhalatoren ist in FR-A-2022212 beschrieben. Der Oberbegriff des Anspruchs 1 beruht auf der Offenbarung dieses Dokumentes.

Der Erfindung liegt daher die Aufgabe zugrunde, einen Inhalator zur Abgabe eines Aerosols zu schaffen, welcher sich durch eine hohe Funktions-Zuverlässigkeit und eine hinsichtlich Ablesbarkeit und Anzeigegenauigkeit verbesserte Dosiermengenzähleinrichtung auszeichnet.

Diese Aufgabe wird erfindungsgemäß durch einen Inhalator mit den im Schutzanspruch 1 angegebenen Merkmalen gelöst.

Dadurch wird es auf vorteilhafte Weise möglich, einen unter fertigungstechnischen Gesichtspunkten günstig herstellbaren Inhalator zu schaffen, bei welchem bedarfsweise jeder einzelne Dosierhub gezählt und angezeigt wird. Neben der damit erreichten zuverlässigen Überwachung der Restfüllmenge eines in dem Inhalator bevorrateten Wirkmediums wird es ferner möglich, die rezeptgemäße Einnahme eines Dosieraerosols besser im Auge zu behalten. Die erfindungsgemäß ausgebildete Dosierzähleinrichtung erlaubt beispielsweise eine von 0 bis ca. 200 aufsteigende Zählung der einzelnen Dosierungen. Alternativ dazu ist es auch möglich, die Zifferanordnung auf den beiden Zählringen derart vorzunehmen, daß eine von der maximalen Hubzahl abwärts laufende Dosierzählung erfolgt. Die Zählung kann beispielsweise von der Zahl 200 abwärts laufend erfolgen, wobei beispielsweise dann, wenn die Resthubzahl ≤ 50 ist, in der Anzeigeeinrichtung eine Farbmarkierung, beispielsweise in Form eines Signalbalkens, sichtbar wird, der dem entsprechenden Benutzer rechtzeitig die Nachbeschaffung eines entsprechenden Inhalators signalisiert.

Die vermittels der erfindungsgemäßen Dosierzähleinrichtung erreichte Verbesserung der Anzeigegenauigkeit erlaubt es, die aus Sicherheitsgründen vorgenommene Überfüllung des Abgabebehälters und damit die ggf. in dem Abgabebehälter verbleibende Wirkstoffrestmenge zu verringern.

Eine besonders zuverlässige Weiterschaltung des ersten Zählrings ist dadurch erreicht, daß der Schaltfinger von der Rotationsachse beabstandet und zu dieser geneigt angeordnet ist. Dadurch wird es auf kinematisch günstige Weise möglich, den Schaltfinger mit zählringseitig vorgesehenen Stellelementen in Eingriff zu bringen und den Schaltfinger anschließend um eine im wesentlichen quer zur Längsrichtung des Schaltfingers verlaufende Kippachse zu kippen. Der Anstellwinkel des Schaltfingers relativ zu einer durch den Zählring definierten Radialebene liegt vorzugsweise im Bereich von 30 bis 60°. Hierbei ergeben sich bei günstigen Kräfteverhältnissen hinreichend große Transportwege des Schaltfingers.

Gemäß einer bevorzugten Ausführungsform der Erfindung ist der Schaltfinger aus einer Ausgangsstellung in eine zu dieser Ausgangsstellung geneigte Endstellung auslenkbar, wobei ein Eingriffsabschnitt des Schaltfingers bereits in der Ausgangsstellung desselben federnd nachgiebig in eine Eingriffsstellung mit dem ersten Zählring gedrängt ist. Dadurch wird auf zuverlässige Weise gewährleistet, daß jeder einzelne Abgabehub des Inhalators durch die Dosierzähleinrichtung erfaßt wird.

Eine im Hinblick auf eine besonders günstige Umsetzung der Abgabehubbewegung des Abgabebehälters des Inhalators in eine zum Betrieb der Dosierzähleinrichtung erforderliche Weiterschaltbewegung vorteilhafte Ausführungsform der Erfindung ist dadurch gegeben, daß ein Armteil vorgesehen ist, das mit dem Schaltfinger gekoppelt ist, wobei das Armteil und der Schaltfinger einen Kniegelenkmechanismus bilden. Dadurch wird bei weiterhin vorteilhaften Kräfteverhältnissen ein vergleichsweise großer Schaltfinger-Transportweg gewährleistet, wodurch es möglich wird, die auf dem ersten Zählring vorgesehenen Ziffern derart weit voneinander beabstandet anzuordnen, daß stets nur eine einzige Ziffer des Zählrings in einem Fensterabschnitt sichtbar ist.

Die zur Zurückstellung des Schaltfingers erforderlichen Rückstellkräfte können auf vorteilhafte Weise durch elastische Verformung des Schaltfingers oder der mit dem Schaltfinger gekoppelten Betätigungselemente aufgebracht werden. Der Schaltfinger besteht in vorteilhafter Weise aus einem Kunststoffmaterial, vorzugsweise einem thermoplastisch umformbaren Kunststoffmaterial.

Eine im Hinblick auf eine zuverlässige Betätigung des Schaltfingers vorteilhafte Ausführungsform der Erfindung ist dadurch gegeben, daß das Armteil und der Schaltfinger über einen Betätigungsabschnitt miteinander verbunden sind. Der Betätigungsabschnitt bildet in vorteilhafter Weise eine einem Schulterabschnitt des Abgabebehälters des Inhalators zugewandte Anlagefläche, die beim Absenken des Abgabebehälters im Rahmen eines Dosierhubs aus einer Ausgangsstellung ausgelenkt, insbesondere im wesentlichen in Axialrichtung des Abgabebehälters, verschoben wird.

Das Armteil und der Schaltfinger sind gemäß einer bevorzugten Ausführungsform der Erfindung einstückig ausgebildet. Vorteilhafterweise ist bei dieser Ausführungsform der Betätigungsabschnitt in einem Verbindungsbereich zwischen Armteil und Schaltfinger vorgesehen. Die Bewegbarkeit von Armteil und Schaltfinger zueinander resultiert im wesentlichen aus der Elastizität des Werkstoffes im Bereich der entsprechenden Übergangsstellen. Die Geometrie dieser Übergangsabschnitte ist derart gewählt, daß zumindest im Rahmen der vorgesehenen Anzahl an Dosierhüben keine erhebliche Materialermüdung oder Querschnittsschwächung in den entsprechenden Verbindungsabschnitten auftritt.

Eine im Hinblick auf eine besonders hohe Funktionszuverlässigkeit der Dosierzähleinrichtung vorteilhafte Ausführungsform der Erfindung ist dadurch gegeben, daß zumindest der erste Zählring, vorzugsweise auch der zweite Zählring, und in besonders vorteilhafte Weise auch der Schaltfinger aus POM gefertigt sind. Die restlichen Komponenten des Inhalators können aus vergleichsweise preiswerten Kunststoffen, wie beispielsweise Polystyrol oder Polyethylen oder sonstigen, vorzugsweise thermoplastisch umformbaren Kunststoffen gebildet sein.

Eine im Hinblick auf eine besonders kompakte und funktionszuverlässige Ausführungsform der Erfindung vorteilhafte Ausgestaltung der Dosierzähleinrichtung ist dadurch gegeben, daß der Schaltfinger, der Betätigungsabschnitt und das Armteil sich im wesentlichen bogenförmig um die Rotationsachse des ersten Zählrings erstrecken. Der Radius dieses Bogens entspricht in vorteilhafter Weise im wesentlichen dem halben Durchmesser der an dem Abgabebehälter ausgebildeten Schulter. Dadurch wird es auf vorteilhafte Weise möglich, die Zählring-Weiterschaltvorrichtung unmittelbar im Bereich des Abgabeventils des Inhalators anzuordnen. Dadurch wird es auf weiterhin vorteilhafte Weise möglich, einen zur Führung des Abgabebehälters des Inhalators vorgesehenen Gehäuseabschnitt durch ein separat ausgebildetes und auf ein Mundstück des Inhalators aufgestecktes Rohrelement auszubilden.

Eine im Hinblick auf eine zuverlässige Zählung der einzelnen Dosierhübe vorteilhafte Ausführungsform der Erfindung ist dadurch gegeben, daß eine Rücklaufsperrvorrichtung vorgesehen ist, die eine Rastklinke aufweist, die spätestens beim Weitertransport des ersten Zählrings um einen Zählschritt in einen entsprechenden zählringseitig ausgebildeten Eingriffsabschnitt eingreift. Alternativ dazu oder auch in Kombination mit einer derartigen Rastklinke ist es möglich, eine Drehung des ersten Zählrings entgegen der Zählring-Weiterschaltrichtung auch durch einen reibschlüssig sperrenden Mechanismus zu verhindern. Die Zählring-Weiterschaltvorrichtung und in vorteilhafter Weise auch die Rücklaufsperrvorrichtung sind gemäß eines besonderen Aspekts der vorliegenden Erfindung derart ausgebildet, daß diese jeden Abgabe- bzw. Dosierhub zählt.

In vorteilhafter Weise ist der Weiterschaltmechanismus derart ausgebildet, daß der erste Zählring auch dann betätigt bzw. weitergedreht wird, wenn ein Abgabehub nur unvollständig ausgeführt wurde. Eine im Hinblick auf eine besonders leichtgängige Ausgestaltung der Rücklaufsperrvorrichtung vorteilhafte Ausführungsform der Erfindung ist dadurch gegeben, daß mehrere Rastklinken vorgesehen sind, die mit entsprechenden am ersten Zählring ausgebildeten Eingriffsabschnitten in Eingriff bringbar sind, wobei die einzelnen Rastklinken derart versetzt ausgebildet sind, daß diese Rastklinken sich jeweils in unterschiedlichen Eingriffszuständen befinden.

Die Länge des Armteils und die Länge des Schaltfingers sowie die Position des Armteils und des Schaltfingers sind in vorteilhafter Weise derart abgestimmt, daß bei einer Verlagerung des Betätigungselementes um eine vorgegebene Wegstrecke der Eingriffsabschnitt des Schaltfingers sich in Umfangsrichtung des ersten Zählrings um eine Wegstrecke verlagert, deren Länge im wesentlichen der Schrittlänge einer umfangsseitig an dem ersten Zählring vorgesehenen Skalierung entspricht. Dadurch wird es auf vorteilhafte Weise möglich, die zur Anzeige jedes einzelnen Zählschrittes erforderliche Nummernkombination eindeutig zur Anzeige zu bringen.

Eine im Hinblick auf eine besonders zuverlässige Zählung und Anzeige der einzelnen Dosierhübe vorteilhafte Ausführungsform der Erfindung ist dadurch gegeben, daß die Skalierung des ersten Zählringes die Ziffern 0, 1, 2... bis 9 trägt, wobei diese Ziffern in gleicher Umfangsteilung am Umfang des ersten Zählrings ausgebildet sind. Diese Ziffern können beispielsweise durch ein Siebdruckverfahren auf den Zählring aufgebracht werden. Gemäß einer besonders bevorzugten Ausführungsform der Erfindung wird die Skalierung jedoch über ein Laser-Beschriftungsverfahren unmittelbar in das Kunststoffmaterial des Zählrings eingebrannt.

Auch die Skalierung des zweiten Zählrings trägt in vorteilhafter Weise die Ziffern 0, 1, 2... bis 9. Die Beschriftung des zweiten Zählrings kann in der gleichen Weise erfolgen wie dies bezüglich des ersten Zählring vorangehend beschrieben wurde.

Eine im Hinblick auf die Zählung einer vergleichsweise großen Hubzahl vorteilhafte Ausführungsform der Erfindung ist dadurch gegeben, daß die Skalierung des zweiten Zählrings die Zahlen 0, 1, 2... bis 20 trägt. Dadurch wird es auf vorteilhafte Weise möglich, mit lediglich zwei Zählringen insgesamt 200 Dosierhübe zu zählen. Insbesondere bei dieser Ausführungsform ist an dem ersten Zählring die Ziffernfolge 0, 1, 2... bis 9 mehrfach, insbesondere 2- oder 3fach, abfolgend angeordnet. Dadurch wird der erforderliche Weiterschalttransportweg des ersten Zählrings erheblich verkürzt.

Die Koppelungsvorrichtung zur Koppelung des zweiten Zählrings mit dem ersten Zählring zur schrittweisen Drehung des zweiten Zählrings gemeinsam mit dem ersten Zählring umfaßt in vorteilhafter Weise eine an dem ersten Zählring ausgebildete, elastisch auslenkbare und mit einem Eingriffsabschnitt des zweiten Zählrings unmittelbar in Eingriff bringbare Zunge. Diese Zunge wird gemäß einer bevorzugten Ausführungsform der Erfindung durch eine relativ zum ersten Zählring stationär angeordnete Anlaufnase ausgelenkt, welche die elastisch auslenkbare Zunge mit dem zweiten Zählring über ein Winkelintervall in Eingriff hält, das exakt dem Weiterschalt-Schrittwinkel des zweiten Zählrings entspricht. Zur Realisierung entsprechenderÜbersetzungsverhältnisse können bedarfsweise mehrere derartige Anlaufnasen an einem relativ zum ersten Zählring stationär angeordneten Bauteil des Inhalators ausgebildet sein. Gegebenenfalls ist es auch möglich, mehrere elastisch auslenkbare und mit dem zweiten Zählring unmittelbar in Eingriff bringbare Zungenelemente an dem ersten Zählring auszubilden.

Eine im Hinblick auf eine besonders zuverlässige Betriebsweise dieser Koppelungsvorrichtung vorteilhafte Ausführungsform des Inhalators ist dadurch gegeben, daß der zweite Zählring einen Lagerungsabschnitt aufweist, der in den ersten Zählring drehbar eingesetzt oder auf den ersten Zählring aufgesetzt ist. Durch Ausbildung entsprechender Umfangsnuten und Eingriffsabschnitte zwischen den beiden Zählringen ist es möglich, die beiden Zählringe zueinander drehbar zu lagern und ein axiales Auseinanderziehen der beiden Zählringe zu verhindern. Die Position des ersten Zählrings relativ zu der Anlaufnase kann auf vorteilhafte Weise durch eine an dem ersten Zählring ausgebildete Umfangsnut eingehalten werden.

Die Drehung des zweiten Zählringes entgegen der Weiterschaltrichtung wird in vorteilhafter Weise durch eine weitere Rücklaufsperrvorrichtung verhindert.

Weitere Einzelheiten und Merkmale der Erfindung ergeben sich aus der nachfolgenden Beschreibung eines bevorzugten Ausführungsbeispiels in Verbindung mit der Zeichnung. Es zeigen:
- Fig. 1: eine Längsschnittansicht durch einen Inhalator mit Mundstück und verschiebbar angeordnetem Abgabebehälter,
- Figuren 2a, 2b und 2c: eine Schemadarstellung zur Erläuterung der Transportmechanik der bei dem Inhalator gem. Fig.1 vorgesehenen Zählring-Weiterschaltvorrichtung,
- Figuren 3a und 3b: Schemadarstellungen zur näheren Beschreibung einer bevorzugten Geometrie eines Schaltfingers, eines Betätigungsabschnittes und eines Armteiles,
- Figuren 4a, 4b und 4c: Axialschnittansichten durch den ersten und den zweiten Zählring zur Erläuterung der Funktionsweise der zur unmittelbaren Koppelung der ersten und zweiten Zählringe vorgesehenen Koppelungseinrichtung,
- Fig. 5: eine Längsschnittansicht durch ein Blendenteil mit integral ausgebildetem Schaltfinger sowie integral ausgebildeter Rücklaufsperrvorrichtung,
- Fig. 6: eine Radialschnittansicht entlang der Linie A-A in Fig. 5 zur Erläuterung des Aufbaus der Rücklaufsperrvorrichtung und
- Fig. 7: eine vereinfachte Seitenansicht des Inhalators gemäß Fig. 1 mit einem im wesentlichen rechteckig ausgebildeten und entlang der Längsachse des Inhalators ausgerichtet verlaufenden Ablesefenster.

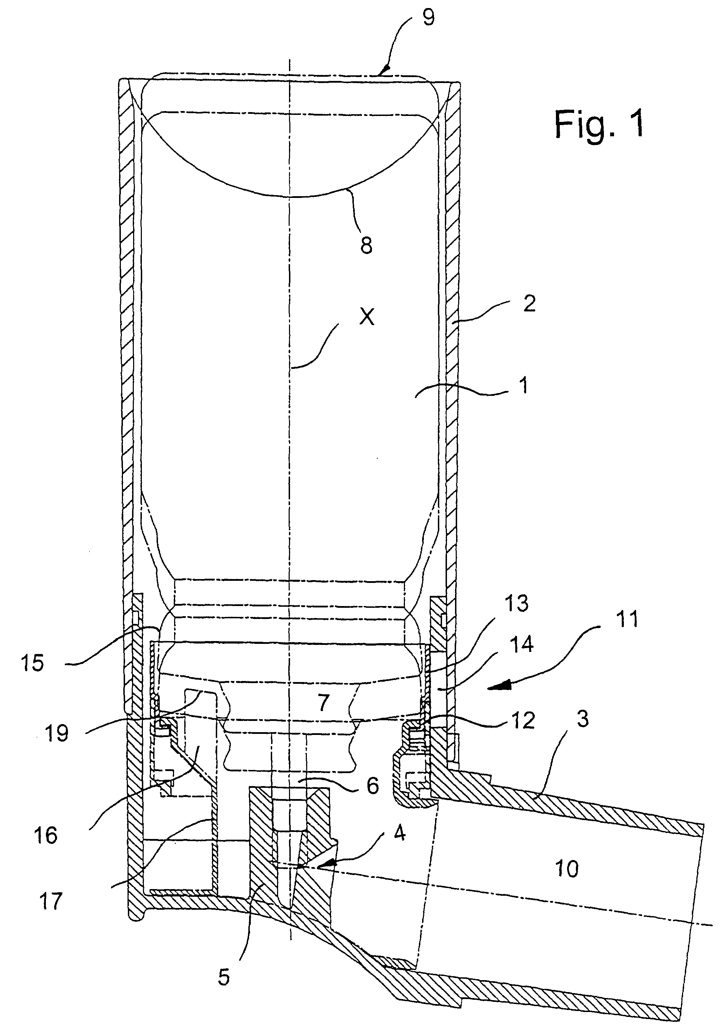

Der in Fig. 1 vereinfacht dargestellte Inhalator zur Abgabe eines Aerosols umfaßt einen entlang seiner Axialrichtung verschiebbar gelagerten Abgabebehälter 1, und einen zur Lagerung des Abgabebehälters 1 vorgesehenen Führungsschaft 2, der passend auf einen entsprechend ausgebildeten Verbindungsabschnitt eines Winkelstückes 3 aufgesetzt ist.

In dem Winkelstück 3 ist eine Sprühdüseneinrichtung 4 angeordnet, die in einem einstückig mit dem Winkelstück 3 ausgebildeten Zapfenabschnitt 5 ausgebildet ist. In diesen Zapfenabschnitt 5 ist ein Abgaberohr 6 des Abgabebehälters 1 in abdichtender Weise eingesetzt. Das Abgaberohr 6 ist in einem Kopfbereich 7 des Abgabebehälters 1 in axialer Richtung nachgiebig aufgenommen und durch eine Federeinrichtung (nicht sichtbar) derart vorgespannt, daß der Abgabebehälter 1 über das Abgaberohr 6 von dem Zapfenabschnitt 5 hinweggedrängt wird.

Im oberen Bereich des Führungsschaftes 2 ist eine Ausmuldung 8 vorgesehen, aus welcher ein Bodenbereich 9 des Abgabebehälters 1 hervorragt. Durch Aufbringen einer Druckkraft auf diesen Bodenbereich 9 kann der Abgabebehälter 1 zu dem Zapfenabschnitt 5 hin verschoben werden, wie dies durch Strichpunktlinien vereinfacht angedeutet ist. Sobald das Abgaberohr 6 an einer entsprechend in dem Zapfenabschnitt 5 ausgebildeten Anlagefläche ansteht, wird das Abgaberohr 6 in den Abgabebehälter 1 hineingedrängt und wirkt dabei mit einem Ventilmechanismus zusammen, über welchen eine vorbestimmte Menge eines in dem Abgabebehälters 1 bevorrateten Mediums aus diesem entströmen und über das Abgaberohr 6 zur Sprühdüseneinrichtung 4 und von dort aus in einen in dem Winkelstück 3 gebildeten Ansaugbereich 10 vordringen kann. Diese nachfolgend als Abgabehub bezeichnete Eintauchbewegung des Abgabebehälters 1 wird durch eine Dosierzähleinrichtung 11 gezählt.

Die Dosierzähleinrichtung 11 umfaßt hierzu einen ersten Zählring 12 und einen zweiten Zählring 13. Der erste Zählring 12 und der zweite Zählring 13 sind zueinander drehbar angeordnet. Der zweite Zählring 13 weist einen Dreh-Lagerungsabschnitt auf, der in einen entsprechenden Öffnungsbereich des ersten Zählrings 12 eingesetzt ist.

Der erste Zählring 12 und der zweite Zählring 13 sind koaxial zur Längsachse X des Abgabebehälters 1 angeordnet. Die beiden Zählringe 12, 13 sind in einem rohrförmigen Abschnitt des Winkelstückes 3 drehbewegbar angeordnet. An den Außenumfangsflächen der beiden Zählringe 12, 13 sind in nachfolgend noch näher erläuterter Weise Ziffern vorgesehen, die über eine Fensteröffnung 14, die in dem Winkelstück 3 ausgebildet ist, ablesbar sind.

Bei der in Fig. 1 dargestellten Ausführungsform des Inhalators befindet sich der zweite Zählring in etwa auf Höhe einer an dem Aufnahmebehälter 1 ausgebildeten Schulter 15. Der zweite Zählring 13 ist hinsichtlich seines Innendurchmessers derart dimensioniert, daß die Schulter 15 des Abgabebehälters 1 über eine der Abgabehublänge entsprechende Wegstrecke in den zweiten Führungsring 13 hinein eindringen kann.

In einem zwischen den beiden Zählringen 12, 13 und dem Kopfbereich 7 des Abgabebehälters verbleibenden Ringraum ist eine Zählring-Weiterschaltvorrichtung 16 vorgesehen, welche den koaxial zum Abgabebehälter 1 angeordneten Zählring 12 bei jedem Abgabehub um einen vorbestimmten Drehwinkel weiterdreht.

Die Zählring-Weiterschaltvorrichtung 16 ist bei der hier dargestellten Ausführungsform einstückig mit einem Blendenteil 17 ausgebildet, das in dem Winkelstück 3 eingesetzt ist. Das Blendenteil 17 bildet einen sich im wesentlichen ringförmig um die Längsachse X erstreckenden Lagerungsabschnitt, an welchem der zweite Zählring 13 gelagert ist.

An dem Blendenteil 17 ist einstückig mit diesem je eine Rücklaufsperrvorrichtung zum Sperren einer Drehbewegung der Zählringe 12,13 in Gegenrichtung vorgesehen.

Das Blendenteil 17 ist ferner mit einer Anlaufnase versehen, die Teil einer im folgenden, ausführlich beschriebenen Koppelungsvorrichtung zur direkten Koppelung der beiden Zählringe 12, 13 bildet.

Die Zählring-Weiterschaltvorrichtung ist innerhalb der beiden Zählringe 12, 13 derart angeordnet, daß ein Betätigungsabschnitt 19 derselben mit der an dem Abgabebehälter 1 ausgebildeten Schulter in Berührungskontakt bringbar ist.

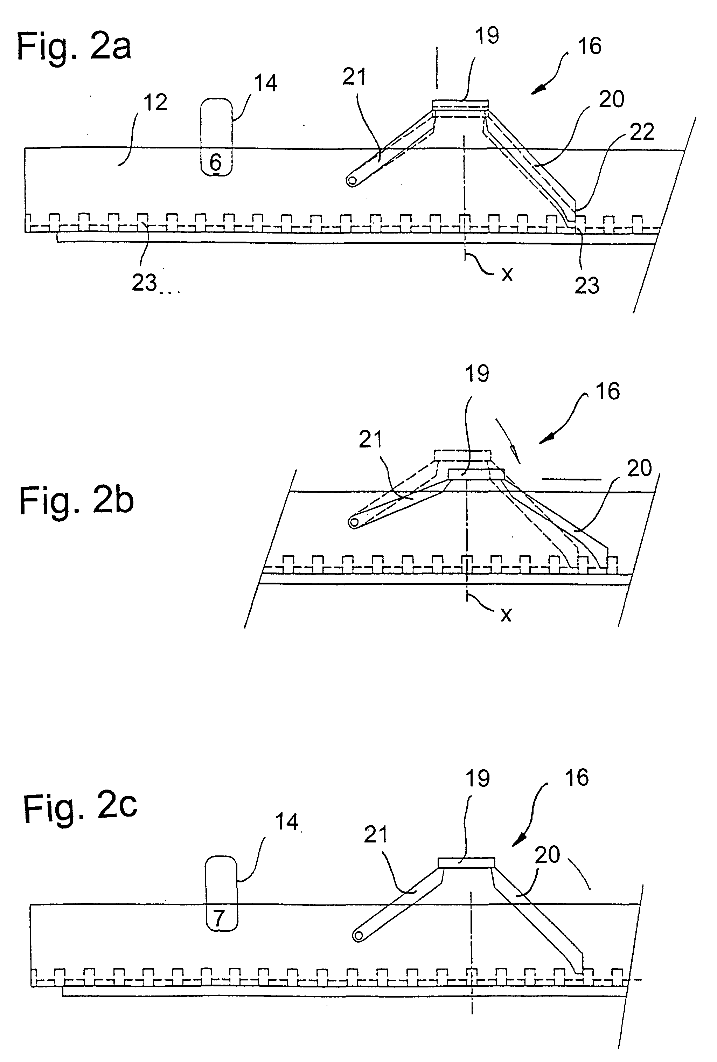

Die Funktionsweise der Zählring-Weiterschaltvorrichtung wird durch die Figuren 2a, 2b und 2c verdeutlicht. Die in den Figuren 2a, 2b und 2c dargestellte Zählring-Weiterschaltvorrichtung 16 umfaßt ein nachfolgend als Schaltfinger 20 bezeichnetes Getriebeglied, das bei der hier dargestellten Ausführungsform über den Betätigungsabschnitt 19 mit einem Armteil 21 gekoppelt ist. Bei der hier dargestellten Ausführungsform sind der Schaltfinger 20, der Betätigungsabschnitt 19 und das Armteil 21 einstückig miteinander ausgebildet und aufgrund der Eigenelastizität des hierbei verwendeten Kunststoffmaterials bewegbar miteinander gekoppelt.

Das Armteil 21 weist ein dem Betätigungsabschnitt 19 abgewandtes, stationäres Ende auf, über welches sich der gesamte durch Armteil 21, Betätigungsabschnitt 19 und Schaltfinger 20 gebildete Gelenkmechanismus in Umfangsrichtung des ersten Zählrings 12 abstützt.

Bei der hier gezeigten Anordnung bilden das Armteil 21 und der Schaltfinger 20 einen Kniehebel-Gelenkmechanismus. Durch allmähliches Niederdrücken des Betätigungsabschnittes entlang der hier angedeutet dargestellten Längsachse X des Abgabebehälters 1 werden das Armteil 21 und der Schaltfinger 20 allmählich in die in Fig. 2 dargestellte Endlage geschwenkt. Bezogen auf eine zu der Achse X senkrechte Projektionsfläche ergibt sich zwischen der Projektion der Position des bewegbaren Endes des Armteils 21 gegenüber der Projektion der Position dieses Endes in der Endstellung ein Versatz. Entsprechend diesem Versatz wird der Betätigungsabschnitt in Umfangsrichtung des ersten Zählrings verlagert. Auch der Schaltfinger 20 wird beim Niederdrücken des Betätigungsabschnittes geschwenkt, so daß sich dabei insgesamt eine Verlängerung der Projektion des Schaltfingers 20 in die zur genannten Längsachse X senkrechte Projektionsfläche ergibt. Nachdem der Schaltfinger 20 mit seinem dem ersten Zählring 12 abgewandten Endabschnitt ebenfalls mit dem Betätigungsabschnitt gekoppelt ist, ergibt sich eine Addition der durch Schwenken des Armteils und des Schaltfingers 20 erreichten Versatzstrecken. Zur Übertragung der entsprechenden in Umfangsrichtung des ersten Zählrings wirksamen Bewegungskomponente auf den ersten Zählring ist der Schaltfinger 20 mit einem Eingriffsabschnitt 22 versehen, der mit zählringseitig ausgebildeten Stellelementen in Eingriff bringbar ist. Durch entsprechende Verlagerung des Eingriffsabschnittes 22 des Schaltfingers 20 in Umfangsrichtung des ersten Zählrings 12 wird aufgrund des Eingriffs mit den Stellelementen 23 der erste Zählring 12 in Umfangsrichtung um eine vorbestimmte Wegstrecke weitertransportiert. Diese vorbestimmte Wegstrecke entspricht im wesentlichen dem Abstand zwischen zwei benachbarten Stellelementen 23. Nachdem der Betätigungsabschnitt 19 durch die an dem Abgabebehälter 1 ausgebildete Schulter in eine untere Endlage verlagert ist, federt beim Zurückfahren des Abgabebehälters 1 in dem Führungsschaft 2 auch der Betätigungsabschnitt 19 aufgrund der Eigenelastizität des Armteils 21 wieder in seine Ausgangsstellung zurück. Bei dieser Rückzugsbewegung überspringt der Schaltfinger 20 bzw. dessen Eingriffsabschnitt das beim nächsten Zählhub mit diesem in Eingriff tretende Stellelement 23 und federt unmittelbar hinter diesem Stellelement 23 wieder in eine Eingriffsstellung. Bei erneutem Niederdrükken des Betätigungsabschnittes 19 schiebt der Eingriffsabschnitt 22 des Schaltfingers das an dem ersten Zählring ausgebildete Stellelement 23 wieder um ein Transportintervall in Umfangsrichtung weiter. Hierbei tritt die nächstfolgende, an der Umfangsfläche des ersten Zählrings 12 vorgesehene Ziffer in die Fensteröffnung 14.

Bei den Darstellungen gemäß Fig. 2a, Fig. 2b und Fig. 2c ist die zur Weiterschaltung des ersten Zählrings vorgesehene Zählring-Weiterschaltvorrichtung in Form einer Abwicklung dargestellt.

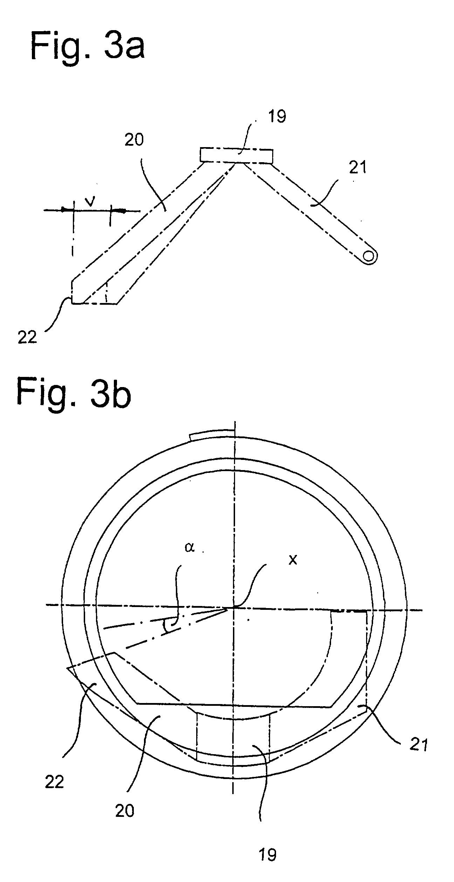

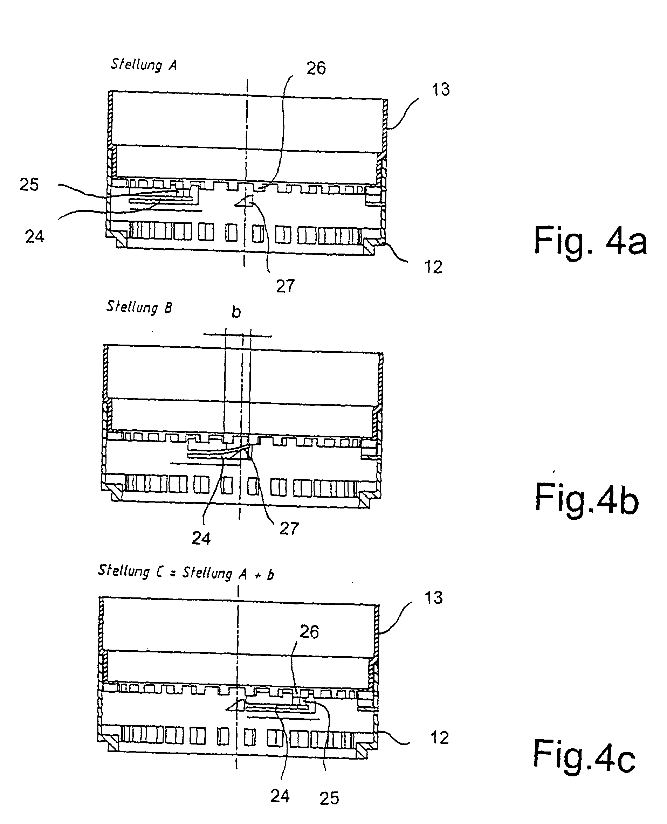

Bei der in Fig. 1 dargestellten Ausführungsform erstreckt sich wie in Fig. 3b dargestellt der durch das Armteil 21, dem Betätigungsabschnitt 19 und den Schaltfinger 20 gebildete Gelenkmechanismus der Zählring-Weiterschaltvorrichtung im wesentlichen bogenförmig um die Längsachse X des Abgabebehälters 1 (in Fig. 3b nicht dargestellt). Durch Niederdrücken des in Fig. 3b dargestellten Betätigungsabschnittes 19 wird ein Versatz des Eingriffsabschnittes 22 des Schaltfingers 21 in Umfangsrichtung erreicht. Durch den Versatz des Eingriffsabschnittes 22 des Schaltfingers 20 in Umfangsrichtung kann der erste Zählring 12 um einen Weiterschaltwinkel α weitergedreht werden. Für den Fall, daß am Außenumfang des ersten Zählrings 12 drei Ziffern-Folgen der Ziffern 0 bis 9 vorgesehen sind, beträgt der Weiterschaltwinkel α ca. 12°. Bei einem derartigen Drehwinkel ergibt sich bereits bei einem dem Durchmesser des Abgabebehälters 1 entsprechenden Durchmessers des ersten Zählrings 1 ein hinreichend großer Abstand zwischen den benachbarten Ziffern, wodurch eine gute Ablesbarkeit der einzelnen Ziffern in der Fensteröffnung 14 gewährleistet ist. Entsprechend der Anzahl der am Umfang des ersten Zählrings 12 abgebildeten Ziffernfolgen wird das Übersetzungsverhältnis zwischen dem ersten Zählring 12 und dem zweiten Zählring 13 bestimmt. Das erforderliche Übersetzungsverhältnis zwischen den beiden Zählringen kann in vorteilhafter Weise durch die nachfolgend in Verbindung mit den Figuren 4a, 4b und 4c beschriebene Koppelungsvorrichtung erreicht werden.

Die in den Figuren 4a, 4b und 4c in unterschiedlichen Koppelungszuständen dargestellte Koppelungsvorrichtung umfaßt ein einstückig mit dem ersten Zählring 12 ausgebildetes Zungenelement 24, das sich bei der Darstellung gemäß Fig. 4a in einer Neutralstellung befindet. An dem Zungenelement 24 ist ein Mitnehmerabschnitt 25 ausgebildet. Dieser Mitnehmerabschnitt 25 ist mit Eingriffsvorsprüngen 26, die einstückig mit dem zweiten Zählring 13 ausgebildet sind, in Eingriff bringbar. Hierzu ist eine am Blendenteil 17 ausgebildete Anlaufnase 27 vorgesehen, die das Zungenelement in eine Kopplungsposition drängt, in welcher der am Ende des Zungenelementes 24 ausgebildete Mitnehmerabschnitt 25 mit dem unmittelbar benachbarten Eingriffsvorsprung 26 des zweiten Zählrings 13 in Eingriff tritt. Sobald ein entsprechender Eingriffszustand hergestellt ist, wird der zweite Zählring 13 gemeinsam mit dem ersten Zählring 12 solange weitergedreht, bis das Zungenelement 24 von der Anlaufnase 27 abfällt und damit der Eingriffszustand zwischen dem Mitnehmerabschnitt 25 und dem entsprechenden Eingriffsvorsprung 26 aufgehoben ist. Sobald dieser Eingriffszustand aufgehoben ist, kann der erste Zählring 12 über eine vorbestimmte Wegstrecke, unabhängig von dem zweiten Zählring 13, weitergedreht werden.

In Fig. 4b ist das Zungenelement 24 durch die in dieser Darstellung nicht sichtbare Anlaufnase 27 in die genannte Koppelungsstellung gedrängt. Die Länge der hier durch den Buchstaben b angegebenen Koppelungsstrecke ist durch die Geometrie der Anlaufnase 27 bestimmt.

In Fig. 4c ist das Zungenelement 24 bereits in seine Ausgangsstellung zurückgefedert und der Eingriff zwischen dem Mitnehmerabschnitt 25 und dem entsprechenden Eingriffsvorsprung 26 des zweiten Zählrings 13 aufgehoben.

Das bei der hier dargestellten Ausführungsform vorgesehene Zungenelement 24 ist einstückig mit dem ersten Zählring 12 als im wesentlichen flachblattfederartiges Bauteil ausgebildet. Im Hinblick auf die erforderlichen mechanischen Eigenschaften des Zungenelementes sowie im Hinblick auf eine besonders hohe Maßhaltigkeit des ersten Zählrings 12 ist dieser vorzugsweise aus POM-Kunststoffmaterial gebildet. Bei der hier dargestellten Ausführungsform sind an der Außenumfangsfläche des zweiten Zählrings 13 die Zahlen 1 bis 20 angeordnet. Durch die beiden Zählringe 12, 13 können in dieser Ausführungsform 200 Dosierungen einzeln gezählt werden. Mit zwei Zählringen ist eine Zählung bis 999 möglich. Unter Verwendung eines dritten Zählringes sind 9.999 Zählungen möglich. Die Koppelungsvorrichtung zur Koppelung eines dritten Zählringes mit dem zweiten Zählring entspricht in ihrem Aufbau in vorteilhafter Weise der zwischen dem ersten Zählring 12 und dem zweiten Zählring 13 vorgesehenen Koppelungsvorrichtung.

Zur Verhinderung einer Drehbewegung des zweiten Zählrings 13 relativ zu dem ersten Zählring 12 entgegen der durch die Koppelungsvorrichtung 24, 25, 26 sowie die Anlaufnase 27 bestimmten Transportrichtung ist für die Zählringe 12 und 13 eine Rücklaufsperrvorrichtung 29 vorgesehen, die mit entsprechenden Rastabschnitten in Eingriff bringbar ist. Bei der hier dargestellten Ausführungsform werden diese Rastabschnitte der Rücklaufsperrvorrichtung auf einfache Weise durch die Eingriffsvorsprünge 23 und 26 gebildet.

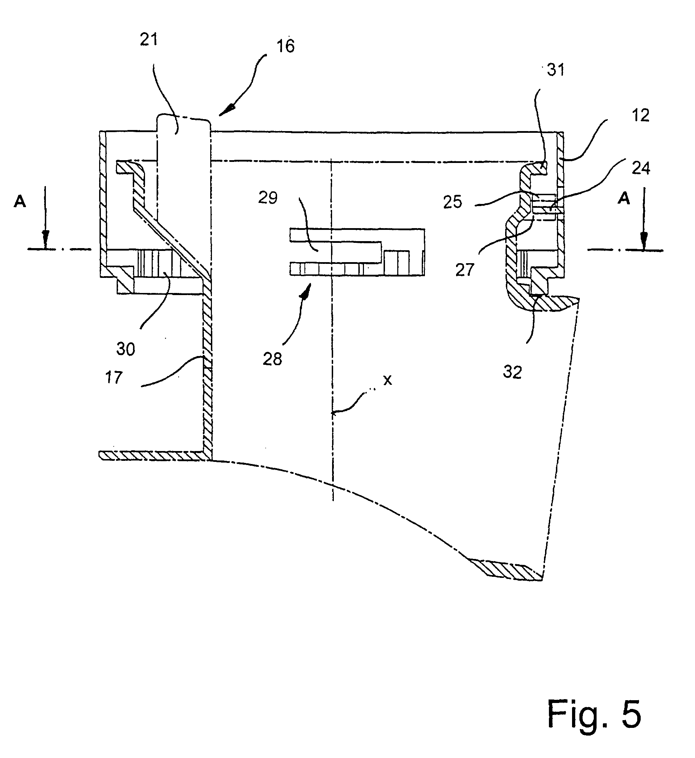

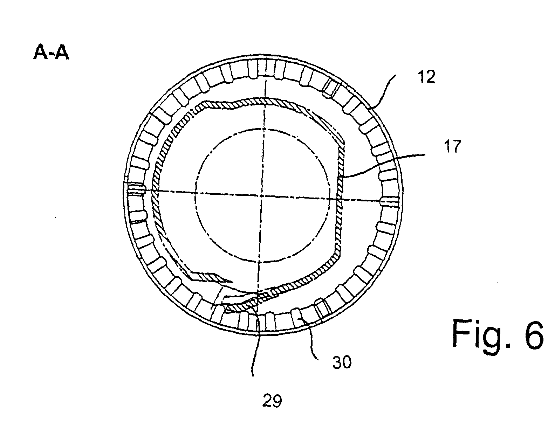

In Fig. 5 sind das bei dem Inhalator gemäß Fig.1 vorgesehene Blendenteil 17 und der darauf aufgesetzte erste Zählring 12 vergrößert dargestellt. Bei der gezeigten Ausführungsform umfaßt die zur Verhinderung einer Drehbewegung des ersten Zählrings 12 entgegen der Zählringweiterschaltrichtung vorgesehe Rücklaufsperrvorrichtung 28 ein einstückig mit dem Blendenteil 17 ausgebildetes Federelement 29, das mit Rastvorsprüngen 30, die am Innenumfang des ersten Zählrings 12 ausgebildet und der Zählringrotationsachse X zugewandt sind, in Eingriff bringbar ist. Die bei dieser Ausführungsform verwirklichte spezielle Geometrie des einstückig mit dem Blendenteil 17 ausgebildeten Federelementes geht aus der in Fig.6 vereinfacht dargestellten Radialschnittansicht entlang der Linie A-A hervor.

Wie aus der Darstellung gemäß Fig.5 weiter ersichtlich, weist das zur direkten Weiterdrehung des zweiten Zählrings 13 (nicht dargestellt) vorgesehene Zungenelement 24 einen flach rechteckförmigen Querschnitt auf und ragt von einer Innenumfangswandung des ersten Zählrings 12 in einen zwischen dem ersten Zählring 12 und dem Blendenteil 17 gebildeten Ringraum hinein. Dieser Ringraum ist in seinem oberen Bereich von einem Anlaufabschnitt 31 begrenzt, welcher den hier nicht dargestellten zweiten Zählring 13 in axialer Richtung lagert. Das hier gezeigte Blendenteil 17 bildet ferner einen zweiten Anlaufabschnitt 32, durch welchen der erste Zählring 12 in axialer Richtung gelagert ist. Im Hinblick darauf, daß sich die ersten und zweiten Zählringe 12,13 gegeneinander in axialer Richtung aufgrund der in Fig.1 gezeigten Schulterstruktur abstützen, sind durch die genannten ersten und zweiten Anlaufabschnitte 31 und 32 die beiden Zählringe 12, 13 insgesamt in axialer Richtung gelagert.

Lediglich in Strichpunktlinien ist in der Schnittansicht gemäß Fig.5 auch die zur Auslenkung des am Endabschnitt des Zungenelementes 24 vorgesehenen Mitnehmerabschnittes 25 vorgesehene Anlaufnase 27 gezeigt. Die einstückig mit dem Blendenteil 17 ausgebildete Zählringweiterschaltvorrichtung 16, insbesondere deren Armteil 21, ist ebenfalls in Strichpunktlinien angedeutet dargestellt.

Die Ausbildung der angesprochenen Filigranstrukturen an einem in einem Hauptgehäuseabschnitt des Inhalators einsetzbaren Blendenteil erweist sich unter fertigungstechnischen Gesichtspunkten als vorteilhaft. Grundsätzlich ist es jedoch auch möglich, auf das Blendenteil 17 zu verzichten und die zur Sperrung der Rücklaufdrehbewegung des ersten Zählrings 12 vorgesehene Rücklaufsperrvorrichtung 28, die Zählringweiterschaltvorrichtung und die entsprechenden Lagerungsabschnitte zur Lagerung der ersten und zweiten Zählringe 12, 13 unmittelbar, d.h. einstückig mit dem Winkelstück 3 auszubilden.

Die in der Schnittansicht gemäß Fig.6 erkennbare Teilung der an dem ersten Zählring 12 vorgesehenen Rastvorsprünge 30 ist derart festgelegt, daß die durch das Federelement 23 gebildete Rücklaufsperrvorrichtung den ersten Zählring 12 nach jedem Zählschritt erneut verrastet. Durch entsprechende Verkleinerung der Rastvorsprünge 30 und Verkleinerung der Abstände zwischen den abfolgenden Rastvorsprüngen ist es möglich, das Rastintervall der Rücklaufsperrvorrichtung zu verkleinern und damit eine Drehbewegung des ersten Zählrings auch dann zu verhindern, wenn dieser erste Zählring 12 nicht um einen vollständigen Zählschritt weitergedreht ist. Durch entsprechende Auslegung der Geometrie des Federelementes 29 kann der durch die Rücklaufsperrvorrichtung 28 erzeugte Drehwiderstand auf einem vergleichsweise geringen Wert gehalten werden.

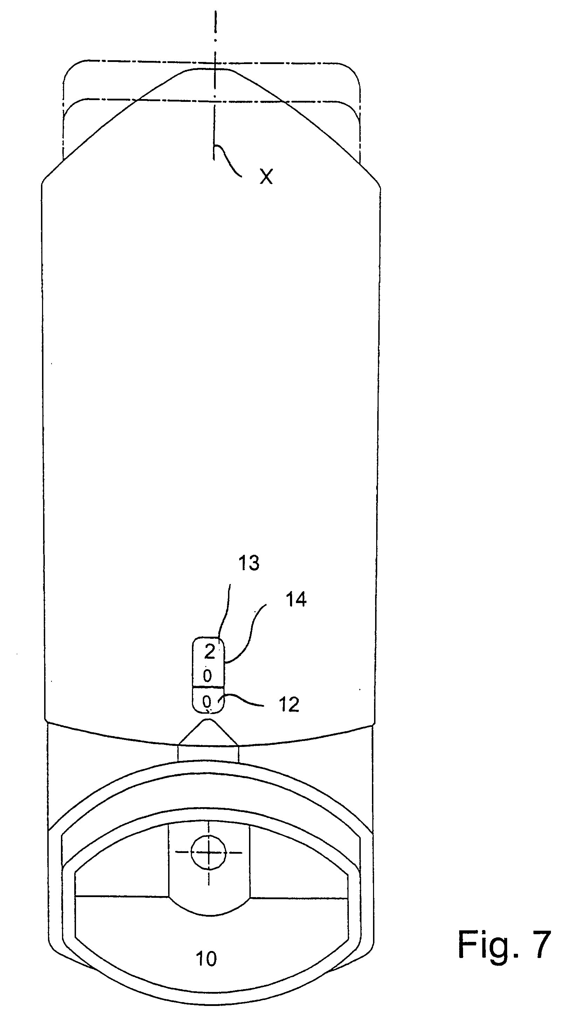

In Fig.7 ist eine Seitenansicht (Seitenansicht von rechts) des in Fig.1 dargestellten Inhalators gezeigt. In der auf seiten des Ansaugbereiches 10 angeordneten Fensteröffnung 14 ist hier die Nummernangabe "200" ablesbar. Diese Nummernangabe wird gebildet durch die über den ersten Zählring 12 in die Fensteröffnung 14 geschobene Ziffer "0" und die über den zweiten Zählring 13 in die Fensteröffnung geschobene Zahl "20". Im Falle einer beispielsweise abwärts laufenden Zählung wird bei der nächsten Betätigung des Inhalators sowohl der erste Zählring 12 als auch der zweite Zählring 13 um ein Winkelintervall (α) um die Längsachse X des Inhalators weitergedreht. Hierbei gelangt über den ersten Zählring 12 die Ziffer "9" und über den zweiten Zählring 13 die Zahl "19" in die Fensteröffnung 14. Damit wird in der Fensteröffnung 14 die Zahl "199" angezeigt. Bei der nächsten Betätigung des Inhalators wird nur der erste Zählring 12 weitergedreht und die Ziffer "8" gelangt in die Fensteröffnung 14. In der Fensteröffnung 14 wird nunmehr die Zahl "198" angezeigt. Im Rahmen der nachfolgenden Dosierhübe wird zunächst nur der erste Zählring 12 jeweils um einen Zählschritt weitergedreht, bis die Ziffer "1" in die Fensteröffnung 14 eintritt und in der Fensteröffnung 14 die Zahl "191" angezeigt wird. Bei der nächstfolgenden Betätigung des Inhalators wird über den in den Fig.4a bis 4c gezeigten Koppelungsmechanismus der erste Zählring 12 unmittelbar mit dem zweiten Zählring gekoppelt und der erste Zählring 12 gemeinsam mit dem zweiten Zählring 13 um einen Zählschritt weitergedreht. Hierbei gelangt die umfangsseitig an dem ersten Zählring vorgesehene Ziffer 0 und die umfangsseitig an dem zweiten Zählring 13 vorgesehene Zahl "19" in die Fensteröffnung 14. In der Fensteröffnung 14 wird nunmehr die Zahl "190" angezeigt. Sobald die Zahl "19" vollständig in die Fensteröffnung 14 gelangt ist, tritt der zur direkten Koppelung der ersten und zweiten Zählringe 12,13 vorgesehene Koppelungsmechanismus wieder in eine Freigabestellung und der erste Zählring 12 wird im Rahmen der nächsten Dosierhübe unabhängig von dem zweiten Zählring 13 gedreht, bis erneut die an dem ersten Zählring 12 vorgesehene Ziffer "1" in die Fensteröffnung 14 gelangt.

Um eine im wesentlichen gleiche Teilung der Ziffernfolgen an dem ersten Zählring 12 und dem zweiten Zählring 13 zu erreichen, ist die Ziffernfolge 0 bis 9 zweifach abfolgend am Außenumfang des ersten Zählrings 12 ausgebildet. Bei einer, bezogen auf die Umfangsrichtung der Zählringe schmaleren Ausgestaltung der Fensteröffnung 14 können an dem zweiten Zählring 13 beispielsweise die Zahlen 0 bis 40 ausgebildet werden. Bei entsprechender Abstimmung des Übersetzungsverhältnisses zwischen dem ersten und dem zweiten Zählring 12,13, beispielsweise durch Vervielfachung der zum Weitertransport des zweiten Zählrings vorgesehenen Anzahl an Anlaufnasen 27 können mit lediglich zwei Zählringen Hubzahlen von 0 bis 400 aufsteigend oder bedarfsweise auch abfallend gezählt werden.

Die Erfindung ist nicht auf das vorgehend beschriebene Ausführungsbeispiel beschränkt. Beispielsweise ist es auch möglich, auf das bei dem vorangehend beschriebenen bevorzugten Ausführungsbeispiel des Inhalators vorgesehene Blendenteil 17 zu verzichten und die entsprechenden Funktionsteile integral mit dem Gehäuse des Inhalators auszubilden. Bedarfsweise können durch den beschriebenen Inhalator auch aerosolfreie Wirkstoffe abgegeben werden. Zur Bereitstellung eines noch größeren Zählbereiches ist es möglich, einen dritten Zählring vorzusehen, der über einen entsprechenden Koppelungsmechanismus mit dem zweiten Zählring koppelbar ist.

## Patentansprüche

1. Inhalator zur Abgabe eines Aerosols, mit:
- einer Dosierzähleinrichtung (11),
- einer Gehäuseeinrichtung (2, 3), und
- einem relativ zu der Gehäuseeinrichtung (2, 3) entlang seiner Längsachse verschiebbar angeordneten Aerosol-Abgabebehälter (1),
- einem ersten Zählring (12), der koaxial zur Längsachse (X) des Aerosol-Abgabebehälters (1) angeordnet ist und um diese Längsachse (X) drehbar ist,
- einem zweiten Zählring (13), der koaxial zu dem ersten Zählring (12) angeordnet und mit diesem direkt koppelbar und relativ zu diesem drehbar ist,
- einer Zählringweiterschaltvorrichtung (16) zur Drehung des ersten Zählringes (12) bei Betätigung des Inhalators, und
- einer Koppelungsvorrichtung (24, 25, 26, 27) zur Koppelung des zweiten Zählringes (13) mit dem ersten Zählring (12), zur schrittweisen Drehung des zweiten Zählringes (13) gemeinsam mit dem ersten Zählring (12), wobei
die Zählringweiterschaltvorrichtung (16) einen Betätigungsabschnitt (19) aufweist, **gekennzeichnet durch** einen mit dem Betätigungsabschnitt (19) gekoppelten Schaltfinger (20), der zur Drehung des zugehörigen ersten Zählringes (12) mit Stellelementen (23) desselben in Eingriff bringbar ist, wobei der Schaltfinger (20) von der Längsachse (X) beabstandet und zu dieser geneigt angeordnet ist.

2. Inhalator nach Anspruch 1 **dadurch gekennzeichnet, daß** der Schaltfinger (20) aus einer Ausgangsstellung in eine zu dieser Ausgangsstellung geneigte Endstellung auslenkbar ist, und in der Ausgangsstellung ein Eingriffsabschnitt des Schaltfingers (20) federnd nachgiebig in eine Eingriffsstellung mit dem ersten Zählring (12) gedrängt ist.

3. Inhalator nach mindestens einem der Ansprüche 1 und 2, **dadurch gekennzeichnet, daß** ein Armteil (21) vorgesehen ist das mit dem Schaltfinger (20) gekoppelt ist, und daß das Armteil (21) und der Schaltfinger (20) einen Kniegelenkmechanismus bilden.

4. Inhalator nach Anspruch 3, **dadurch gekennzeichnet, daß** das Armteil (21) elastisch nachgiebig ausgebildet ist und an einem stationären Teil (17) insbesondere einem mit der Gehäuseeinrichtung (2, 3) gekoppelten Teil abgestützt ist.

5. Inhalator nach mindestens einem der Ansprüche 3 und 4, **dadurch gekennzeichnet, daß** das Armteil (21) und der Schaltfinger (20) über den Betätigungsabschnitt (17) miteinander verbunden sind.

6. Inhalator nach mindestens einem der Ansprüche 3 bis 5, **dadurch gekennzeichnet, daß** das Armteil (21) und der Schaltfinger (20) einstückig ausgebildet sind.

7. Inhalator nach mindestens einem der Ansprüche 3 bis 6, **dadurch gekennzeichnet, daß** das Armteil (21) und der Schaltfinger (20) aus einem Kunststoffmaterial, POM, ausgebildet sind.

8. Inhalator nach mindestens einem der Ansprüche 3 bis 7, **dadurch gekennzeichnet, daß** der Betätigungsabschnitt (19) in eine zur Rotationsachse des ersten Zählringes (12) im wesentlichen parallele Richtung geführt ist, und daß das Armteil (21) ein Federelement bildet das den Schaltfinger (20) in seine Ausgangsstellung drängt.

9. Inhalator nach mindestens einem der Ansprüche 1 bis 8, **dadurch gekennzeichnet, daß** der Betätigungsabschnitt (19) eine Anlagefläche bildet die mit einer Schulter (19) des Aerosol-Abgabebehälters (1) in Anlage bringbar ist.

10. Inhalator nach mindestens einem der Ansprüche 3 bis 9, **dadurch gekennzeichnet, daß** der Schaltfinger (20), der Betätigungsabschnitt (19) und das Armteil (21) sich im wesentlichen bogenförmig um die Längsachse (X) des ersten Zählringes (12) erstrecken.

11. Inhalator nach mindestens einem der Ansprüche 1 bis 10, **dadurch gekennzeichnet, daß** eine Rücklaufsperrvorrichtung (28) vorgesehen ist, zum Verhindern einer Drehbewegung des ersten Zählrings (12) entgegen der Zählrichtung.

12. Inhalator nach Anspruch 11, **dadurch gekennzeichnet, daß** die Rücklaufsperrvorrichtung (28) eine Rastklinke aufweist.

13. Inhalator nach mindestens einem der Ansprüche 3 bis 12, **dadurch gekennzeichnet, daß** die Länge des Armteiles (21) und die Länge des Schaltfingers (20) sowie die Position des Armteiles (21) und des Schaltfingers (2a) derart abgestimmt sind, daß bei einer Verlagerung des Betätigungselementes (19) um eine vorgegebene Wegstrecke der Eingriffsabschnitt (22) des Schaltfingers (20) sich in Umfangsrichtung des ersten Zählringes (12) um eine Wegstrecke verlagert deren Länge im wesentlichen der Schrittlänge einer umfangsseitig an dem ersten Zählring (12) vorgesehenen Skalierung entspricht.

14. Inhalator nach mindestens einem der Ansprüche 1 bis 13, **dadurch gekennzeichnet, daß** die Skalierung des ersten Zählringes (12) die Ziffern 0, 1, 2... bis 9 trägt.

15. Inhalator nach mindestens einem der Ansprüche 1 bis 14, **dadurch gekennzeichnet, daß** die Skalierung des zweiten Zählringes (13) die Ziffern 0, 1, 2,... bis 9 trägt.

16. Inhalator nach mindestens einem der Ansprüche 1 bis 14, **dadurch gekennzeichnet, daß** die Skalierung des zweiten Zählringes (13) die Zahlen 0, 1, 2,...9, 10, 11,... 19 und 20 trägt.

17. Inhalator nach Anspruch 14, **dadurch gekennzeichnet, daß** die Ziffernfolge 0, 1...9 am Umfang des ersten Zählringes (12) mehrfach; insbesondere dreimal abfolgend vorgesehen ist.

18. Inhalator nach mindestens einem der Ansprüche 1 bis 17, **dadurch gekennzeichnet, daß** der zweite Zählring (13) einen Lagerungsabschnitt aufweist, der in den ersten Zählring (12) drehbar eingesetzt oder auf diesen aufgesetzt ist.

19. Inhalator nach mindestens einem der Ansprüche 1 bis 18, **dadurch gekennzeichnet, daß** die Koppelungsvorrichtung (24, 25, 26, 27) ein Mitnehmerelement aufweist das mit dem ersten Zählring (12) gekoppelt ist.

20. Inhalator nach Anspruch 19, **dadurch gekennzeichnet, daß** das Mitnehmerelement durch ein elastisch nachgiebiges Zungenelement (24) gebildet ist, das einstückig mit dem ersten Zählring (12) ausgebildet ist.

21. Inhalator nach Anspruch 19 oder 20, **dadurch gekennzeichnet, daß** der zweite Zählring (13) einen Verzahnungsabschnitt aufweist der mit dem Zungenelement (24) in Eingriff bringbar ist.

22. Inhalator nach einem der Ansprüche 19 bis 21, **dadurch gekennzeichnet, daß** die Koppelungsvorrichtung eine stationäre Anlauf-Nase (27) aufweist, zum Auslenken des Zungenelements (24) derart, daß dieses über eine, einem Zählschritt entsprechende Umfangslänge mit dem zweiten Zählring (13) in Eingriff tritt und den zweiten Zählring (13) um diese Umfangslänge weiterdreht.

23. Inhalator nach Anspruch 22, **dadurch gekennzeichet,** daß drei in Umfangsrichtung gleichmäßig beabstandete stationäre Anlauf-Nasen (27) vorgesehen sind derart, daß der zweite Zählring (13) bei einem vollständigen Umlauf den zweiten Zählring (13) um drei Zählschritte weiterdreht.

## Claims

1. Inhaler for dispensing an aerosol with:
- a dosage counting system (11),
- a casing part (2, 3) and
- an aerosol dispensing container (1) disposed to be displaceable along its longitudinal axis relative to the casing part (2, 3),
- a first counting ring (12), which is disposed coaxially to the longitudinal axis (X) of the aerosol dispensing container (1) and is rotatable around this longitudinal axis (X),
- a second counting ring (13), which is disposed coaxially to the first counting ring (12) and may be coupled directly to this and is rotatable relative to this,
- a counting ring advancing device (16) for rotating the first counting ring (12) upon operation of the inhaler, and
- a coupling device (24, 25, 26, 27) for coupling the second counting ring (13) to the first counting ring (12), for stepped rotation of the second counting ring (13) jointly with the first counting ring (12),
wherein the counting ring advancing device (16) has an operating section (19), **characterised by** a shift finger (20) coupled to the operating section (19), which, for rotation of the associated first counting ring (12), may be brought into engagement with positioning elements (23) thereof, wherein the shift finger (20) is spaced from the longitudinal axis (X) and arranged inclined relative to this.

2. Inhaler according to Claim 1, **characterised in that** the shift finger (20) may be deflected out of a starting position into an end position inclined relative to this starting position, and in the starting position an engagement section of the shift finger (20) is pushed in a resiliently flexible manner into a position of engagement with the first counting ring (12).

3. Inhaler according to at least one of Claims 1 and 2, **characterised in that** an arm portion (21) is provided, which is coupled to the shift finger (20), and that the arm portion (21) and the shift finger (20) form a toggle joint mechanism.

4. Inhaler according to Claim 3, **characterised in that** the arm portion (21) is designed to be resiliently flexible and is supported on a stationary part (17) in particular a part coupled to the casing part (2, 3).

5. Inhaler according to at least one of Claims 3 and 4, **characterised in that** the arm portion (21) and the shift finger (20) are connected to one another via the operating section (17).

6. Inhaler according to at least one of Claims 3 to 5, **characterised in that** the arm portion (21) and the shift finger (20) are constructed in one piece.

7. Inhaler according to at least one of Claims 3 to 6, **characterised in that** the arm portion (21) and the shift finger (20) are constructed from a plastic material, POM.

8. Inhaler according to at least one of Claims 3 to 7, **characterised in that** the operating section (19) is directed into a direction essentially parallel to the rotational axis of the first counting ring (12), and that the arm portion (21) forms a spring element which pushes the shift finger (20) into its starting position.

9. Inhaler according to at least one of Claims 1 to 8, **characterised in that** the operating section (19) forms a contact surface which can be brought into abutment with a shoulder (19) of the aerosol dispensing container (1).

10. Inhaler according to at least one of Claims 3 to 9, **characterised in that** the shift finger (20), the operating section (19) and the arm portion (21) extend essentially in an arc shape around the longitudinal axis (X) of the first counting ring (12).

11. Inhaler according to at least one of Claims 1 to 10, **characterised in that** a return stop device (28) is provided to prevent a rotational movement of the first counting ring (12) contrary to the counting direction.

12. Inhaler according to Claim 1, **characterised in that** the return stop device (28) has a stop catch.

13. Inhaler according to at least one of Claims 3 to 12, **characterised in that** the length of the arm portion (21) and the length of the shift finger (20) as well as the position of the arm portion (21) and of the shift finger (2a) are coordinated in such a manner that when the operating element (19) shifts a predetermined distance, the engagement section (22) of the shift finger (20) shifts in the circumferential direction of the first counting ring (12) by a distance, the length of which corresponds essentially to the step length of a scale provided circumferentially on the first counting ring (12).

14. Inhaler according to at least one of Claims 1 to 13, **characterised in that** the scale of the first counting ring (12) bears the numbers 0, 1, 2 .. to 9.

15. Inhaler according to at least one of Claims 1 to 14, **characterised in that** the scale of the second counting ring (13) bears the numbers 0, 1, 2 .. to 9.

16. Inhaler according to at least one of Claims 1 to 14,
**characterised in that** the scale of the second counting ring (13) bears the numbers 0, 1, 2 ..9, 10 11, .. 19 and 20.

17. Inhaler according to Claim 14, **characterised in that** the numerical sequence 0, 1...9 is provided several times on the circumference of the first counting ring (12), in particular three times in succession.

18. Inhaler according to at least one of Claims 1 to 17, **characterised in that** the second counting ring (13) has a bearing section, which is rotatably inserted into the first counting ring (12) or is attached onto this.

19. Inhaler according to at least one of Claims 1 to 18, **characterised in that** the coupling device (24, 25, 26, 27) has an entrainment element which is coupled to the first counting ring (12).

20. Inhaler according to Claim 19, **characterised in that** the entrainment element is formed by an elastically flexible tongue element (24) which is constructed in one piece with the first counting ring (12).

21. Inhaler according to Claim 19 or 20, **characterised in that** the second counting ring (13) has a toothed section which may be brought into engagement with the tongue element (24) .

22. Inhaler according to one of Claims 19 to 21, **characterised in that** the coupling device has a stationary run-up lug (27) for deflecting the tongue element (24) in such a manner that this moves into engagement with the second counting ring (13) over a circumferential length corresponding to a counting step and further rotates the second counting ring (13) by this circumferential length.

23. Inhaler according to claim 22, **characterised in that** there are provided three stationary run-up lugs (27) equally spaced circumferentially such that with a complete rotation the second counting ring (13) further rotates the second counting ring by three counting steps.

## Revendications

1. Inhalateur pour délivrer un aérosol, comprenant,
- un dispositif de comptage des doses (11),
- un dispositif de boîtier (2, 3),
- un récipient fournisseur d'aérosol (1), pouvant coulisser par rapport au dispositif de boîtier (2, 3) le long de son axe longitudinal,
- une première bague de comptage (12) coaxiale à l'ax longitudinal (X) du récipient fournisseur d'aérosols et pouvant tourner autour de cet axe,
- une seconde bague de comptage (13), coaxiale à la première, (12) et pouvant tourner par rapport à celle-ci et lui être accouplée directement,
- un dispositif d'avancement successif (16) pour faire tourner la première bague de comptage (12) quand on actionne l'inhalateur,
- un dispositif d'accouplement (24, 25, 26, 27) pour accoupler la deuxième bague de comptage (13) à la première (12) et pour faire tourner ensemble et pas à pas les deux bagues,
**caractérisé en ce que**
le dispositif d'avancement successif (16) présente une partie d'actionnement (19), **caractérisée par** un doigt de commutation (20) qui lui est accouplé et qui, pour faire tourner la première bague correspondante (12) peut venir en prise avec des éléments de positionnement (23) de celle-ci, le doigt de commutation (20) étant situé à une certaine distance de l'axe longitudinal (X) et incliné vers lui.

2. Inhalateur selon la revendication 1,
**caractérisé en ce que**
le doigt de commutation (20) peut être dévié pour passer dune position initiale à une position finale inclinée par rapport à la position initiale et dans laquelle une partie de prise du doigt de commutation (20) est poussée en flexion élastique dans une position où il est en prise avec la première bague de comptage (12).

3. Inhalateur selon Tune des revendications 1 et 2,
**caractérisé en ce que**
il est prévu un bras (21) accouplé au doigt (20) en formant avec celui-ci un mécanisme à genouillère.

4. Inhalateur selon Tune des revendications 1 à 3,
**caractérisé en ce que**
le bras (21) est flexible élastiquement et en appui sur une partie stationnaire (17), en particulier une partie accouplée au dispositif de boîtier (2, 3).

5. Inhalateur selon Tune des revendications 3 et 4,
**caractérisé en ce que**
le bras (21) et le doigt de commutation (20) sont reliés par une partie d'actionnement (17).

6. Inhalateur selon Tune des revendications 3 à 5,
**caractérisé en ce que**
le bras (21) et le doigt de commutation (20) forment une seule pièce.

7. Inhalateur selon Tune des revendications 3 à 6,
**caractérisé en ce que**
le bras (21) et le doigt de commutation (20) sont réalisés en une matière plastique, en particulier de l'oxyde de polyméthylène.

8. Inhalateur selon Tune des revendications 3 à 7,
**caractérisé en ce que**
la partie d'actionnement (19) est guidée selon une direction essentiellement parallèle à l'axe de rotation de la première bague de comptage (12) et le bras (21) constitue un élément élastique que le doigt de commutation (20) pousse à sa position initiale.

9. Inhalateur selon Tune des revendications 1 à 8,
**caractérisé en ce que**
la partie d'actionnement (19) forme une portée de contact qui peut être amenée en contact avec un épaulement (15) du récipient fournisseur d'aérosol (1).

10. Inhalateur selon Tune des revendications 3 à 9,
**caractérisé en ce que**
le doigt de commutation (20), la partie d'actionnement (19) et le bras (21) sont disposés essentiellement en arc de cercle autour de l'axe longitudinal (X) de la première bague de comptage (12).

11. Inhalateur selon Tune des revendications 1 à 10,
**caractérisé en ce que**
il est prévu un dispositif anti-retour (28) pour empêcher la première bague de comptage (12) de tourner dans le sens opposé au comptage.

12. Inhalateur selon la revendication 11,
**caractérisé en ce que**
le dispositif anti-retour (28) comprend un cliquet.

13. Inhalateur selon Tune des revendications 3 à 12,
**caractérisé en ce que**
la longueur du bras (21) et la longueur du doigt de commutation (20) ainsi que la position de ce bras et de ce doigt sont accordées de manière qu'un déplacement de l'élément d'entrainement (19) présentant une longueur de course donnée à l'avance, fait effectuer à la partie de prise (22) du doigt de commutation (20), selon la direction périphérique de la première bague de comptage (12), une course dont la longueur correspond essentiellement à celle du pas dune échelle prévue à la périphérie de la première bague de comptage (12).

14. Inhalateur selon Tune des revendications 1 à 13,
**caractérisé en ce que**
l'échelle de la première bague de comptage (12) porte les chiffres 0, 1, 2,..., 9.

15. Inhalateur selon Tune des revendications 1 à 14,
**caractérisé en ce que**
l'échelle de la seconde bague de comptage (13) porte les chiffres 0, 1, 2,..., 9.

16. Inhalateur selon Tune des revendications 1 à 14,
**caractérisé en ce que**
l'échelle de la seconde bague de comptage (13) porte les chiffres 0, 1, 2,..., 9, 10, 11,..., 19 et 20.

17. Inhalateur selon Tune des revendications 1 à 14,
**caractérisé en ce que**
la suite des chiffres 0, 1, 2,..., 9, est portée plusieurs fois sur la périphérie de la première bague de comptage (12), en particulier trois fois.

18. Inhalateur selon Tune des revendications 1 à 17,
**caractérisé en ce que**
la seconde bague de comptage (13) présente une partie de palier, montée en rotation dans la première bague de comptage (12) ou posée sur celle-ci.

19. Inhalateur selon Tune des revendications 1 à 18,
**caractérisé en ce que**
le dispositif d'accouplement (24, 25, 26, 27) présente un élément d'entraînement qui est accouplé à la première bague de comptage (12).

20. Inhalateur selon la revendication 1 à 19,
**caractérisé en ce que**
l'élément d'entraînement est constitué par un élément à languette (24), flexible élastiquement et monobloc avec la première bague de comptage (12).

21. Inhalateur selon Tune des revendications 19 ou 20,
**caractérisé en ce que**
la seconde bague de comptage (13) présente une partie dentée qui peut venir en prise avec l'élément à languette (24).

22. Inhalateur selon Tune des revendications 19 à 21,
**caractérisé en ce que**
le dispositif d'accouplement présente une dent d'accostage (27) stationnaire, pour dévier l'élément à languette (24) de manière que celui-ci vienne, sur une longueur périphérique correspondant à un pas de comptage, en prise avec la seconde bague de comptage (13) et la fasse tourner de cette longueur périphérique.

23. Inhalateur selon Tune des revendications 19 à 22,
**caractérisé en ce que**
il est prévu trois dents d'accostage stationnaires, également espacées en direction périphérique, de sorte que la première bague de comptage (12), quand elle effectue un tour complet, fait tourner la seconde bague de comptage (13) de trois pas de comptage.
